(19) Europäisches Patentamt
European Patent Office
Office européen des brevets

(11) Publication number: **0 197 488 A2**

# (12) EUROPEAN PATENT APPLICATION

(21) Application number: **86104419.6**

(22) Date of filing: **01.04.86**

(51) Int. Cl.⁴: **C07D 211/90**, C07D 401/14, C07D 403/14, C07D 401/12, A01N 43/40, A01N 43/42

(30) Priority: **01.04.85 JP 68649/85**

(43) Date of publication of application: **15.10.86 Bulletin 86/42**

(84) Designated Contracting States: **AT BE CH DE FR GB IT LI NL SE**

(71) Applicant: **Eisai Co., Ltd.**
**6-10, Koishikawa 4-chome Bunkyo-ku**
**Tokyo 112(JP)**

(72) Inventor: **Sugimoto, Hachiro**
**3073-13, Kashiwada Ushikumachi**
**Inashiki-gun Ibaraki(JP)**
Inventor: **Higurashi, Kunizo**
**19-13, Kasuga 4-chome Yatabe-machi**
**Tsukuba-gun Ibaraki(JP)**
Inventor: **Shoji, Tadao**
**57-7, Houyoudai Kukizakimachi**
**Inashiki-gun Ibaraki(JP)**
Inventor: **Adachi, Hideyuki Lions'mansion**
**Kashiiguusandou 403 2-28, Kashii 1-chome**
**Higashi-ku Fukuoka-shi, Fukuoka 813(JP)**
Inventor: **Daiku, Yoshiharu**
**15-3, Umezono 2-chome Sakuramura**
**Niihari-gun Ibaraki(JP)**

(74) Representative: **Lehn, Werner, Dipl.-Ing. et al**
**Hoffmann, Eitle & Partner Patentanwälte**
**Arabellastrasse 4 (Sternhaus)**
**D-8000 München 81(DE)**

## (54) 1,4-Dihydropyiridine derivative, a process for preparing the same, a pharmaceutical composition as well as the use thereof.

(57) A novel 1,4-dihydropyridine derivative is disclosed having the formula:

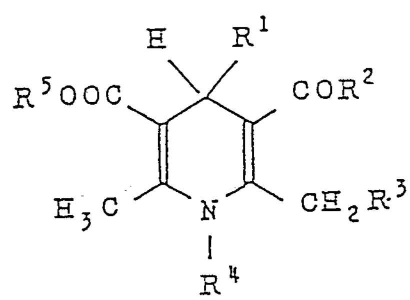



Rank Xerox

This derivative and pharmacologically acceptable salts thereof are effective for the treatment and prevention of cardiopathy and antihypertensive and coronary vasodilating action.

There is also described a process for the preparation of the claimed 1,4-dihydropyridine derivative, a pharmacological composition containing the same and the use thereof.

# 1,4-Dihydropyridine derivative, a process for preparing the same, a pharmaceutical composition as well as the use thereof

The present invention relates to novel 1,4-dihydropyridine derivatives having an excellent pharmacological action.

Particularly, it relates to a 1,4-dihydropyridine derivative represented by the general formula:

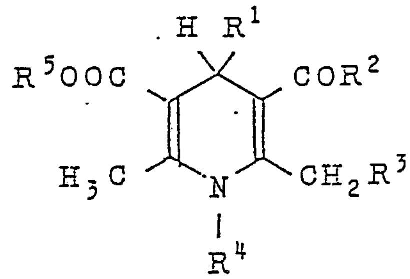

(I)

wherein $R^1$ stands for an aryl or heteroaryl group which may be substituted with a lower alkyl, lower alkoxy, halogen, nitro, trifluoromethyl or cyano group or may form an alkylenedioxy group together with adjacent carbon atom constituting an aryl group; $R^2$ stands for a lower alkoxy group, a group represented by the general formula:

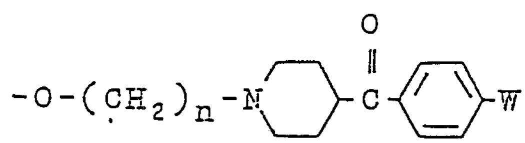

(wherein n stands for a positive number of 2 to 6 and W stands for a hydrogen or halogen atom or a lower alkoxy group) or a group represented by the general formula:

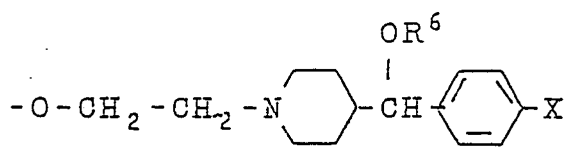

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^4$ stands for a hydrogen atom, a lower alkyl or phenylaklyl group or a group represented by the formula $-CH_2-L$ [wherein L stands for a group of $-OR^7$ (wherein $R^7$ stands for a lower alkyl group) or a group represented by the general formula:

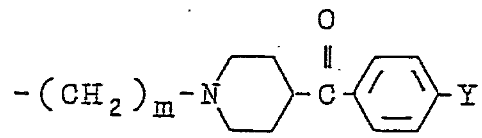

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

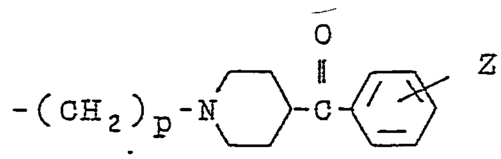

(wherein p stands for a positive number of 1 to 6 and Z stands for a hydrogen or halogen atom or a lower alkoxy group) or a group represented by the general formula:

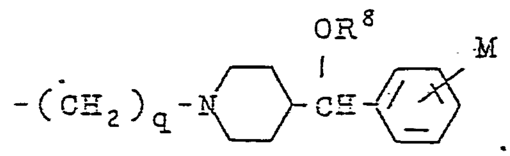

(wherein q stands for a positive number of 1 to 6 and M stands for a hydrogen or halogen atom or a lower alkoxy group),

or a pharmaceutically acceptable salt thereof, a process for the preparation thereof and a medicine containing it as an active principle.

In the above definition of the compound (I) according to the present invention, the term "lower alkyl" refers to straight-chain or branched alkyl groups having 1 to 6 carbon atoms, such as methyl, ethyl, n-propyl, isopropyl, n-butyl, isobutyl, 1-methylpropyl, tert-butyl, n-pentyl, 1-ethylpropyl, isoamyl or n-hexyl group. Further, the terms "lower alkoxy group" and "lower alkoxy lower alkyl group" refer to groups each derived from the above lower alkyl groups.

Representative examples of the aryl group in the definition of $R^1$ include phenyl and naphthyl groups.

$R^2$ and $R^3$ may together form a ring. Particularly, the present invention includes compounds represented by the formula:

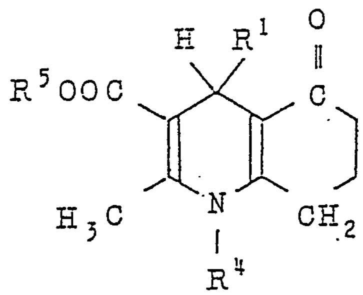

or

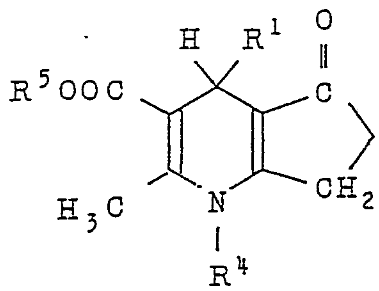

The "halogen" in the definition of W, X, Y, Z or M refers to fluorine, bromine, iodine and chlorine.

The compound (I) of the present invention can be converted into its acid addition salt by reacting it with a pharmaceutically acceptable inorganic or organic acid. Examples of the inorganic acid include hydrochloric, hydrobromic, hydroiodic, sulfuric and phosphoric acids, while those of the organic acid include maleic, fumaric, succinic, acetic, malonic, citric, benzoic, oxalic, and methanesulfonic acids.

The compound (I) of the present invention is thought owing to its structural characteristics to be present as optical isomers based on the asymmetric carbon atom at position 3 of the 1,4-dihydropyridine ring. The present invention includes all of the isomers.

The compound of the present invention can be prepared by various methods, among which representative methods are as follows:

Peparation method A [for compounds represented by the formula (I) wherein $R^4$ is H]

The following three compounds are reacted to obtain an objective compound.

**1** A compound represented by the general formula (II)

$R^1$-CHO (II)

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom constituting an aryl group.

Examples of the compound include benzaldehyde derivatives represented by the general formula:

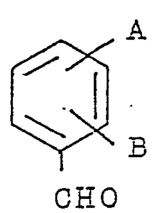

(II′)

wherein A and B may be the same or different from each other and each stands for a lower alkyl, lower alkoxy, halogen, nitro, trifluoromethyl or cyano group or the both may together form an alkylenedioxy group,

and compounds represented by the formula:

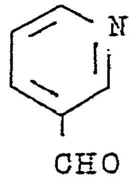 (II″) , 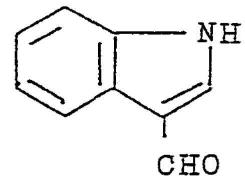 (II‴)

or

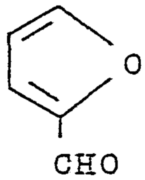 (II⁗)

2 An acetoacetate derivative represented by the general formula:

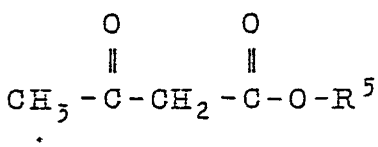 (III)

wherein $R^5$ stands for a lower alkyl or lower alkoxy lower alkyl group, a group represented by the general formula:

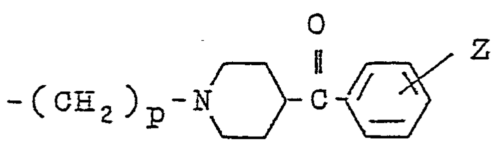

(wherein p stands for a positive number of 1 to 6 and Z stands for a hydrogen or halogen atom or a lower alkoxy group) or a group represented by the general formula:

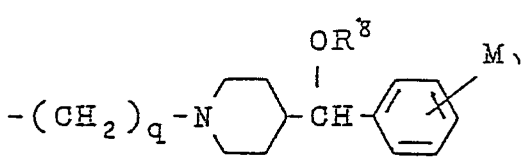

(wherein q stands for a positive number of 1 to 6 and M stands for a hydrogen or halogen atom or a lower alkoxy group).

3 An enaminocarboxylate derivative represented by the general formula:

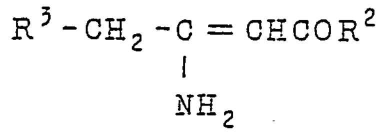 (IV)

wherein $R^3$ stands for a hydrogen atom or a 4-p-(fluorobenzoyl)-1-piperidyl group; $R^2$ stands for a lower alkoxy group, a group represented by the general formula:

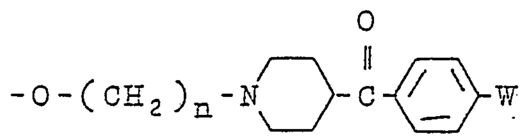

(wherein n stands for a positive number of 2 to 6 and W stands for a hydrogen or halogen atom or a lower alkoxy group) or a group represented by the formula:

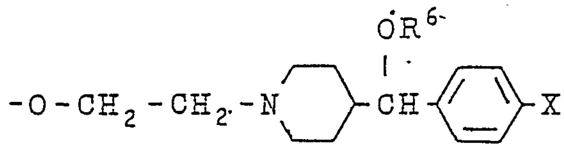

(wherein $R^6$ stands for a hydrogen atom or a nitro group and X stands for a hydrogen or halogen atom or a lower alkoxy group); alternatively, $R^2$ and $R^3$ may together form a ring.

The above three components are reacted in the presence of an alcohol preferably selected from among methanol, ethanol, n-propanol, isopropanol, butanol and the like, preferably at a temperature of 50 to 150°C.

The amounts of the components **1** and **3** to be used in the reaction are preferably each 1.0 to 2.0 mol per mol of the component **2**.

An acetoacetate derivative represented by the general formula (III) to be used as a starting material in the above reaction can be prepared by, for example, the following method.

An alcohol derivative represented by the general formula:

HO-$R^5$ (V)

wherein $R^5$ is as defined above is reacted with a diketene in the presence or absence of a solvent to obtain an objective acetoacetate derivative. Preferred examples of the solvent include benzene, toluene and xylene. It is preferred that an alcohol derivative (V) is reacted with a diketene in a molar ratio of between 1 : 1 and 1 : 3. The reaction temperature is preferably 0 to 150°C.

<u>Preparation method B</u> [for compounds represented by the formula (I) wherein $R^4$ is H]

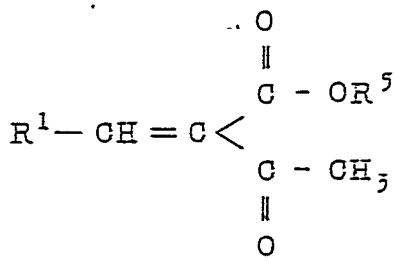

(VI)

wherein $R^1$ and $R^5$ are as defined above

+

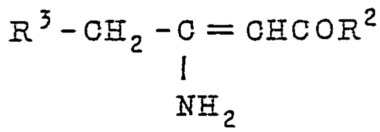

(IV)

wherein $R^2$ and $R^3$ are as defined above

↓

(I)

That is to say, a benzylideneacetoacetate derivative represented by the general formula (VI) is reacted with an enaminocarboxylate derivative (IV) to obtain an objective compound (I).

This reaction is carried out generally at a temperature of 50 to 150°C in the presence of an alcohol solvent such as methanol, ethanol, n-butanol, isopropanol or butanol. It is preferred that a benzylideneacetoacetate derivative (VI) is reacted with an enaminocarboxylate derivative (IV) in a molar ratio of between 1 : 1 and 1 : 2.

A benzylideneacetoacetate derivative (VI) to be used as a starting material in this method can be prepared by reacting a compound represented by the general formula (II) with a compound represented by the general formula (III) according to known methods, for example, a method described in Organic Synthesis, Collective Vol. 4, p.408 - (1963).

Preparation Method C

(for compounds represented by the general formula (I) wherein $R^4$ stands for a hydrogen atom, a lower alkyl or phenylalkyl group or a group represented by the formula $-CH_2-L$ [wherein L stands for a group of $-OR^7$resented by (wherein $R^7$ stands for a lower alkyl group) or a group represented by the general formula:

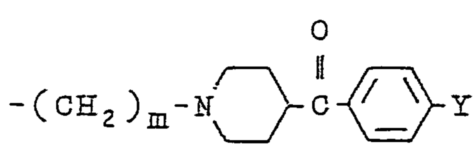

(wherein m stands for a positive number of 0 to 5 and Y stands for a hydrogen or halogen atom or a lower alkoxy group)}.

The following three components are condensed to obtain an objective compound.

1 A compound represented by the general formula:

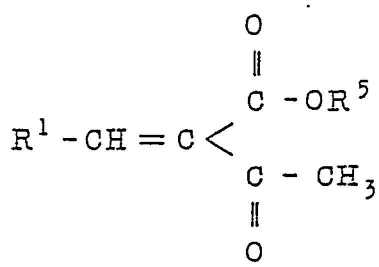

(VI)

wherein $R^1$ and $R^5$ are as defined above.

**2** A compound represented by the general formula:

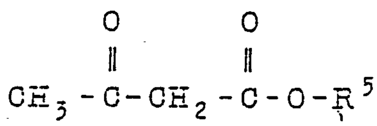

(III)

wherein $R^5$ is as defined above

or

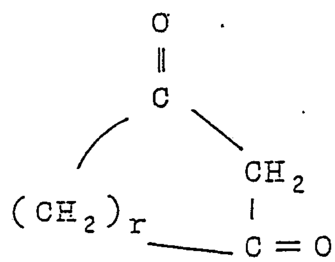

(VII)

wherein r stands for a positive number of 2 to 4.

**3** An aqueous ammonia (VIII) or a compound represented by the general formula:

$H_2N-(CH_2)_s-R^9$ (IX)

wherein s stands for a positive number of 0 to 6 and $R^9$ stands for a hydrogen atom or a lower alkyl, phenylalkyl, lower alkoxy or phenyl group, a group represented by the formula

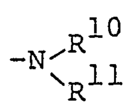

(wherein $R^{10}$ and $R^{11}$ may be the same or different from each other and each stands for a lower alkyl group) or a group represented by the formula

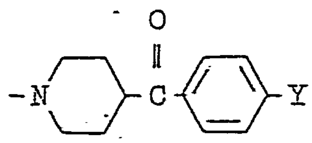

(wherein Y stands for a hydrogen or halogen atom or a lower alkoxy group).

The above condensation of components **1**, **2** and **3** is carried out in the presence of a solvent at a temperature of 50 to 150°C to obtain an objective compound. It is preferred to use about 1.0 to 2.0 mol of component **2** and about 1.0 to 10 mol of component **3** per mol of component **1** in the condensation. The solvent is preferably selected from among alcohols and particular examples thereof include methanol, ethanol, n-butanol, isopropanol and butanol.

Now, representative compounds of the present invention will be described to give a better understanding of the present invention. Of course, these representative compounds do not restrict the present invention, but are for the above purpose.

- 2-[4-(p-fluorobenzoyl)-1-piperidyl]ethyl isopropyl 2,6-dimethyl-4-(o,m-dichlorophenyl)-1,4-dihyrdropyridine-3,5-dicarboxylate
- 2-[4-(p-fluorobenzoyl)-1-piperidyl]ethyl propyl 2,6-dimethyl-4-(o,m-dichlorophenyl)-1,4-dihydropyridine-3,5-dicarboxylate
- 2-[4-(p-fluorobenzoyl)-1-piperidyl]ethyl methoxyethyl 2,6-dimethyl-4-(o,m-dichlorophenyl)-1,4-dihydropyridine-3,5-dicarboxylate
- 2-[4-(p-chlorobenzoyl)-1-piperidyl]ethyl benzyl 2,6-dimethyl-4-(o,m-dichlorophenyl)-1,4-dihydropyridine-3,5-dicarboxylate
- 2-[4-(benzoyl)-1-piperidyl]ethyl methyl 2,6-dimethyl-4-(m-nitrophenyl)-1,4-dihydropyridine-3,5-dicarboxylate
- 2-[4-(p-chlorobenzoyl)-1-piperidyl]ethyl methyl 2,6-dimethyl-4-(m-nitrophenyl)-1,4-dihydropyridine-3,4-dicarboxylate
- 2-[4-(p-bromobenzoyl)-1-piperidyl]ethyl methyl 2,6-dimethyl-4-(m-nitrophenyl)-1,4-dihydropyridine-3,5-dicarboxylate
- 2-[4-(p-methoxybenzoyl)-1-piperidyl]ethyl methyl 2,6-dimethyl-4-(m-nitrophenyl)-1,4-dihydropyridine-3,5-dicarboxylate
- 2-[4-(p-fluorobenzoyl)-1-piperidyl]ethyl methyl 1-ethyl-2,6-dimethyl-4-(o-nitrophenyl)-1,4-dihydropyridine-3,5-dicarboxylate
- 2-[4-(p-fluorobenzoyl)-1-piperidyl]ethyl methyl 1-benzyl-2,6-dimethyl-4-(o-nitrophenyl)-1,4-dihydropyridine-3,5-dicarboxylate
- 2-[4-(p-fluorobenzoyl)-1-piperidyl]ethyl ethoxymethyl 1-benzyl-2,6-dimethyl-4-(o-nitrophenyl)-1,4-dihydropyridine-3,5-dicarboxylate
- 2-[4-(p-fluorobenzoyl)-1-piperidyl]ethyl methyl 1-{2-[4-(p-fluorobenzoyl)-1-piperidyl]ethyl}-2,6-dimethyl-4-(o-nitrophenyl)-1,4-dihydropyridine-3,5-dicarboxylate
- 4-(m-nitrophenyl)-3-ethoxycarbonyl-5-methoxycarbonyl-6-methyl-2-[4-(p-fluorobenzoyl)-1-piperidyl]-ethoxymethyl-1,4-dihydropyridine
- 2-[4-(p-fluorobenzoyl)-1-piperidyl]ethyl methyl 2,6-dimethyl-4-(m-nitrophenyl)-1,4-dihydropyridine-3,5-dicarboxylate
- 2-[4-(p-fluorobenzoyl)-1-piperidyl]ethyl methyl 2,6-dimethyl-4-(2',3'-dichlorophenyl)-1,4-dihydropyridine-3,5-dicarboxylate
- 2-[4-(p-fluorobenzoyl)-1-piperidyl]ethyl ethyl 2,6-dimethyl-4-(2',3'-dichlorophenyl)-1,4-dihydropyridine-3,5-dicarboxylate
- 2-[4-(p-fluorobenzoyl)-1-piperidyl]ethyl 2-methoxyethyl 2,6-dimethyl-4-(m-nitrophenyl)-1,4-dihydropyridine-3,5-dicarboxylate
- 2-[4-(p-fluorobenzoyl)-1-piperidyl]ethyl methyl 2,6-dimethyl-4-(o-nitrophenyl)-1,4-dihydropyridine-3,5-dicarboxylate
- 2-[4-(p-fluorobenzoyl)-1-piperidyl]ethyl ethyl 2,6-dimethyl-4-(o-nitrophenyl)-1,4-dihydropyridine-3,5-dicarboxylate
- 2-[4-(p-fluorobenzoyl)-1-piperidyl]ethyl ethyl 2,6-dimethyl-4-(o-nitrophenyl)-1,4-dihydropyridine-3,5-dicarboxylate

• 2-[4-(p-fluorobenzoyl)-1-piperidyl]ethyl methyl 2,6-dimethyl-4-(m-trifluoromethylphenyl)-1,4-dihydropyridine-3,5-dicarboxylate

• 3-[4-(p-fluorobenzoyl)-1-piperidyl]proxyl methyl 2,6-dimethyl-4-(o-nitrophenyl)-1,4-dihydropyridine-3,5-dicarboxylate

• 6-[4-(p-fluorobenzoyl)-1-piperidyl]hexyl methyl 2,6-dimethyl-4-(o-nitrophenyl)-1,4-dihydropyridine-3,5-dicarboxylate

• 2-[4-(hydroxy-p-fluorophenylmethyl)-1-piperidyl]-ethyl methyl 2,6-dimethyl-4-(m-nitrophenyl)-1,4-dihydropyridine-3,5-dicarboxylate

• 2-[4-(nitroxy-p-fluorophenylmethyl)1-piperidyl]-ethyl methyl 2,6-dimethyl-4-(m-nitrophenyl)-1,4-dihydropyridine-3,5-dicarboxylate

• 2-[4-(p-fluorobenzoyl)-1-piperidyl]ethyl methyl 2,6-dimethyl-4-(m,p-dichlorophenyl)-1,4-dihydropyridine-3,5-dicarboxylate

• 2-[4-(p-fluorobenzoyl)-1-piperidyl]ethyl methyl 2,6-dimethyl-4-(o-chlorophenyl)-1,4-dihydropyridine-3,5-dicarboxylate

• 2-[4-(p-fluorobenzoyl)-1-piperidyl]ethyl methyl 2,6-dimethyl-4-(o-bromophenyl)-1,4-dihydropyridine-3,5-dicarboxylate

• 2-[4-(p-fluorobenzoyl)-1-piperidyl]ethyl methyl 2,6-dimethyl-4-(p-chlorophenyl)-1,4-dihydropyridine-3,5-dicarboxylate

• 2-[4-(p-fluorobenzoyl)-1-piperidyl]ethyl methyl 2,6-dimethyl-4-(o,p-dichlorophenyl)-1,4-dihydropyridine-3,5-dicarboxylate

• 2-[4-(p-fluorobenzoyl)-1-piperidyl]ethyl methyl 2,6-dimethyl-4-(2'-chloro-5'-nitrophenyl)-1,4-dihydropyridine-3,5-dicarboxylate

• 2-[4-(p-fluorobenzoyl)-1-piperidyl]ethyl methyl 2,6-dimethyl-4-(2',6'-dichlorophenyl)-1,4-dihydropyridine-3,5-dicarboxylate

• 2-[4-(p-fluorobenzoyl)-1-piperidyl]ethyl methyl 2,6-dimethyl-4-(m-chlorophenyl)-1,4-dihydropyridine-3,5-dicarboxylate

• 2-[4-(p-fluorobenzoyl)-1-piperidyl]ethyl methyl 2,6-dimethyl-4-(o-cyanophenyl)-1,4-dihydropyridine-3,5-dicarboxylate

• 2-[4-(p-fluorobenzoyl)-1-piperidyl]ethyl methyl 2,6-dimethyl-4-naphthyl-1,4-dihydropyridine-3,5-dicarboxylate

• 2-[4-(p-fluorobenzoyl)-1-piperidyl]ethyl methyl 2,6-dimethyl-4-(3-pyridyl)-1,4-dihydropyridine-3,5-dicarboxylate

• 2-[4-(p-fluorobenzoyl)-1-piperidyl]ethyl methyl 2,6-dimethyl-4-(2'-chloro-4',5'-methylenedioxyphenyl)-1,4-dihydropyridine-3,5-dicarboxylate

• 2-[4-(p-fluorobenzoyl)-1-piperidyl]ethyl methyl 2,6-dimethyl-4-(m-nitro-p-chlorophenyl)-1,4-dihydropyridine-3,5-dicarboxylate

• 2-[4-(p-fluorobenzoyl)-1-piperidyl]ethyl methyl 2,6-dimethyl-4-(2-furyl)-1,4-dihydropyridine-3,5-dicarboxylate

• 2-[4-(p-fluorobenzoyl)-1-piperidyl]ethyl methyl 2,6-dimethyl-4-(3-indolyl)-1,4-dihydropyridine-3,5-dicarboxylate

• 2-[4-(hydroxy-p-fluorophenylmethyl)-1-piperidyl]-ethyl ethyl 2,6-dimethyl-4-(m-chlorophenyl)-1,4-dihydropyridine-3,5-dicarboxylate

• 2-methyl-3-methoxycarbonyl-4-(m-nitrophenyl)-5-ethoxycarbonyl-6-[4-(p-fluorobenzoyl)-1-piperidyl]-methyl-1,4-dihydropyridine

• 2-[4-(p-fluorobenzoyl)-1-piperidyl]ethyl ethyl 2,6-dimethyl-4-(2',3'-dichlorophenyl)-1,4-dihydropyridine-3,5-dicarboxylate

• 2-[-4-(p-fluorobenzoyl)-1-piperidyl]ethyl ethyl 2,6-dimethyl-4-(o-chlorophenyl)-1,4-dihydropyridine-3,5-dicarboxylate

• 2-[4-(p-fluorobenzoyl)-1-piperidyl]ethyl methyl 2,6-methyl-4-(o-fluorophenyl)-1,4-dihydropyridine-3,5-dicarboxylate

• 2-[4-(p-fluorobenzoyl)-1-piperidyl]ethyl 2-[4-p-fluorobenzoyl)-1-piperidyl]ethyl 2,6-dimethyl-4-(m-nitrophenyl)-1,4-dihydropyridine-3,5-dicarboxylate

• 2-methyl-3-[2-(4-p-fluorobenzoyl)-1-piperidyl]-ethoxycarbonyl-4-(m-nitrophenyl)-5(1H, 4H, 6H, 7H,

8H)-pentahydroquinolinone

- 2-[4-(p-fluorobenzoyl)-1-piperidyl]ethyl 2-methoxyethyl 2,6-dimethyl-4-(m-nitrophenyl)-3,5-dicarboxylate

The 1,4-dihydropyridine derivatives of the present invention exhibits excellent hypotensive and coronary vasodilating actions.

Up to this time, some 1,4-dihydropyridine derivatives have been used as a medicine for treatment of cardiopathy, brain circulation failure or hypertension. Representatively, nifedipin[4-(2'-nitrophenyl)-2,6-dimethyl-3,5-dicarbomethoxy-1,4-dihydropyridine] and nicaldipin{methyl 2-[methyl-(phenylmethyl)amino]ethyl 1,4-dihydro-2,6-dimethyl-4-(3-nitrophenyl)-3,5-pyridinedicarboxylate} are known as such a medicine. However, these compounds exhibit a relatively short duration of action, thus being significantly problematic in persistency. A medicine for circulatory disease must generally be used repeatedly for a long time owing to characteristics of this disease, so that the development of a medicine for treatment of hypertension or cardiopathy exhibiting a persistent and stable action has been desired.

The inventors of the present invention have long studied on 1,4-dihydropyridine derivative free from the above disadvantage and have found that a 1,4-dihydropyridine derivative represented by the formula (I) is free from the disadvantage and is very useful as a medicine for treatment of cardiopathy, brain circulation failure, hypertension or the like. The present invention has been accomplished on the basis of this finding.

The present invention relates to a 1,4-dihydropyridine derivative represented by the general formula:

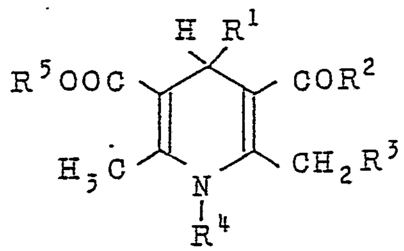

(I)

wherein R' stands for an aryl or heteroaryl group which may be substituted with a lower alkyl, lower alkoxy, halogen, nitro, trofluoromethyl or cyano group or may form an alkylenedioxy group together with an adjacent carbon atom constituting an aryl group; $R^2$ stands for a lower alkoxy group, a group represented by the general formula:

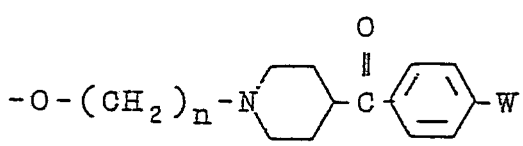

(wherein n stands for a positive number of 2 to 6 and W stands for a hydrogen or halogen atom or a lower alkoxy group) or a group represented by the general formula:

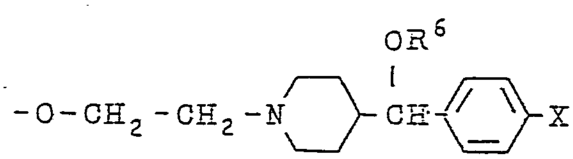

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

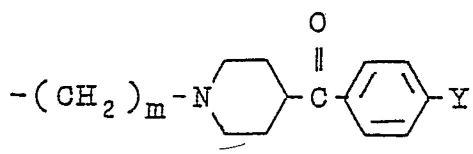

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

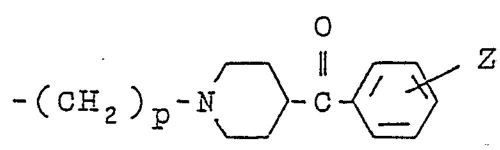

(wherein p stands for a positive number of 1 to 6 and Z stands for a hydorgen or halogen atom or a lower alkoxy group) or a group represented by the general formula:

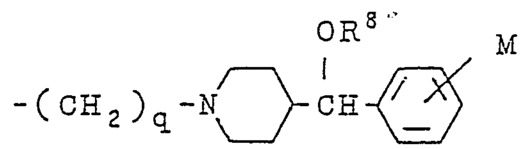

(wherein q stands for a positive number of 1 to 6 and M stands for a hydrogen or halogen atom or a lower alkoxy group),

or its pharmaceutically acceptable salt.

The compound (I) of the present invention is characterized by exhibiting not only persistent hypotensive and coronary vasodilating actions based on calcium antagonism but also remarkable serotonin-blocking (5-$HT_2$) and $\alpha_1$-blocking action which no 1,4-dihydropyridine derivatives of the prior art can exhibit.

The compound of the present invention is useful as a medicine for treatment or prevention of cardiopathy with an enhanced thrombotic power such as hypertension, angina pectori or myocardial infarction.

That is to say, an object of the present invention is to provide a novel 1,4-dihydropyridine derivative which exhibits persistent coronary vasodilating and hypotensive actions and is useful as a medicine based on these actions, a process for the preparation thereof and a medicine containing it as an active principle.

Now, Experimental Examples will be given to illustrate the effects of the compound of the present invention in more detail.

Experimental Example 1

Antihypertensive action

(1) Experimental procedure

A spontaneously hypertensive male rat (SHR) in a non-narcotized state was examined for systolic pressure at a tail artery with a sphygmomanometer (KN-209 type made by Natsume Works).

Each test compound was suspended on 0.5% solution of methylcellulose and adminstered per os with a gastrosonde.

Nifedipin and nicaldipin were used as a control.

(2) Test compounds

Compound A: 2-[4-(p-fluorobenzoyl)-1-piperidyl]ethyl ethyl 2,6-dimethyl-4-(o-nitrophenyl)-1,4-dihydropyridine-3,5-dicarboxylate hydrochloride

Compound B: 2-[-4(p-fluorobenzoyl)-1-piperidyl]ethyl methyl 2,6-dimethyl-4-(m-nitrophenyl)-1,4-dihydropyridine-3,5-dicarboxylate hydrochloride

Compound C: 2-[4-(p-fluorobenzoyl)-1-piperidyl]ethyl ethyl 2,6-dimethyl-4-(o,m-dichlorophenyl)-1,4-dihydropyridine-3,5-dicarboxylate hydrochloride

Compound D: 2-[4-(p-fluorobenzoyl)-1-piperidyl]ethyl ethyl 2,6-dimethyl-4-(o-chlorophenyl)-1,4-dihydropyridine-3,5-dicarboxylate hydrochloride

(3) Results

The obtained results are shown in Table 1. Table 1 shows the maximum degree of blood pressure drop after the oral administration of 10 mg/kg of a test compound (ΔmmHg), duration of action and changes in heart rate (Δ heart rate/min).

Table 1

| Test compound | Hypotensive action | | Heart rate Δ heart rate/min |
|---|---|---|---|
| | systolic pressure Δ mmHg | duration of action Δ hours | |
| Compound A | -39.3 | 7 | -50.7 |
| B | -58.0 | 8 or above | +16.6 |
| C | -70.3 | 8 or above | +38.5 |
| D | -33.5 | 8 or above | -18.8 |
| nifedipin | -40.6 | 4 | +23.5 |
| nicaldipin | -37.3 | 4 | +15.3 |

Experimental Examination 2

Coronary vasodilating action and action on blood pressure or heart rate

(1) Experimental procedure

Mongrel dogs (male and female) were narcotized with pentobarbital sodium and examined for blood pressure at a femoral artery with a pressure transducer (MPU-0.5A made by Nippon Koden) and for heart rate with a heart rate tachometer. Further, the flow rate of coronary blood was determined with an electromagnetic blood-flow meter - (MFV-1100 made by Nippon Koden) whose probe

was jointed to a left-lotating plate. The recording was carried out with a pen oscillograph. Each test compound was dissolved in a 50% solution of polyethylene glycol and injected intravenously.

(2) Results

The obtained results are shown in Table 2, wherein the coronary vasodilating action is shown in terms of ratio (percentage) of flow rate for 15 minutes after the intravenous administration of 10 μg/kg of a test compound to that before the administration.

Table 2

| Test compound | Flow rate of coronary blood Δ ml/15 min. | Average blood pressure Δ % | Heart rate Δ % |
|---|---|---|---|
| Compound B | +232 ± 26 | -26 ± 5.5 | +0.8 ± 3.0 |
| C | +362 ± 126 | -20 ± 3.0 | -2.7 ± 2.7 |
| D | +350 ± 211 | -23 ± 7.8 | +8.7 ± 4.9 |
| nifedipin | +133 ± 42 | -24 ± 7.0 | +4.3 ± 4.3 |
| nicaldipin | +103 ± 51 | -39 ± 8.3 | -2.0 ± 2.0 |

It is clear from the above results that the compound of the present invention exhibits hypotensive and coronary vasodilating actions. Accordingly, it is useful as a medicine for treatment or prevention of hypertension, brain circulation failure, and cardiopathy such as ischemic heart disease (for example, angina pectori or myocardial infarction).

Particularly, the compound of the present invention is characterized by exhibiting a long duration of action, thus being very valuable in this point.

The dose of the compound of the present invention to be administered to a patient of such a disease is varied depending upon kind or degree of disease, kind of a compound or age of a patient, though not particularly limited. Generally, the compound of the present invention is administered with a dose of about 1 to 1000 mg, preferably about 5 to 300 mg, more preferably 5 to 60 mg per adult and a day orally or parenterally. The dose is preferably administered in 2 to 4 instalments a day. The administration form may be powder, fine granule, granule, tablet, capsule, injection or the like.

The compound of the present invention exhibits a remarkably low toxicity, thus being very safe.

The compound of the present invention can be converted into a medicine by using an ordinary carrier according to an ordinary method.

For example, a solid medicine for oral administration can be prepared by mixing a principal agent with a filler and, if necessary, a binder, a disintegrating agent, a lubricant, a coloring agent, a corrigent or the like and converting the obtained mixture into tablet, coated tablet, granule, powder, capsule or the like according to an ordinary method.

Examples of the filler include lactose, corn starch, sucrose, glucose, sorbitol, crystalline cellulose and silicon dioxide, while those of the binder include polyvinyl alcohol, polyvinyl ether, ethylcellulose, methylcellulose, acacia, tragacanth, gelatin, shellac, hydroxypropylcellulose, hydroxypropylstarch and polyvinylpyrrolidone. Examples of the disintegrating agent include starch, agar, gelatin powder, crystalline cellulose, calcium carbide, sodium hydrogen carbonate, calcium citrate, dextrin and pectin. Examples of the lubricant include magnesium stearate, talc, polyethylene glycol, silica and hardened vegetable oil. The coloring agent may be any one which is premitted to be added to drugs. Examples of the corrigent include cocoa

powder, menthol, aromatic acid, mentha oil, borneol and cinnamon bark powder. The tablet and glanule may of course be coated with sugar, gelatin or other suitable substances.

A medicine for injection can be prepared by, if necessary, mixing a principal agent with pH adjuster, buffer, stabilizer, solubilizing agent or the like and converting the resulting mixture into a medicine for injection according to an ordinary method.

The following Formulation Examples 1 and 2 will describe the particular examples of a medicine containing the compound of the present invention, wherein the "active principal" refers to 2-[4-(p-fluorobenzoyl)-1-piperidyl]ethyl ethyl 2,6-dimethyl-4-(o-fluorophenyl)-1,4-dihydropyridine-3,5-dicarboxylate.

Formulation Example 1 Tablet

| | |
|---|---|
| active principal | 10.0 parts by weight |
| lactose | 53.5 |
| microcrystalline cellulose | 18.0 |
| corn starch | 18.0 |
| calcium stearate | 0.5 |

According to an ordinary method, the above components were mixed and converted into a granule. The granule was compression-molded into a tablet having a weight of 100 mg.

Formulation Example 2 Capsule

| | |
|---|---|
| active principal | 10.0 parts by weight |
| lactose | 70.0 |
| corn starch | 20.0 |

A capsule having a weight of 100 mg was prepared by the use of the above formulation according to an ordinary method.

Now, Examples of the present invention will be described below, though the present invention is not limited by them.

Example 1

2-[4-(p-Fluorobenzoyl-1-piperidyl]ethyl methyl 2,6-dimethyl-4-(m-nitrophenyl)-1,4-dihydropyridine-3,5-dicarboxylate hydrochloride

8.3 g of m-nitrobenzaldehyde, 2.9 g of methyl 3-aminocrotonate, 3.8 g of 2-[4-(p-fluorobenzyl)-1-piperidyl]ethyl acetoacetate and 100 ml of isopropanol were mixed and heated under reflux for 5 hours.

The reaction mixture was concentrated under a reduced pressure. The residue was purified by silica gel chromatograph (with an eluent consisting of chloroform and ethanol in a ratio by volume of 20 to 1) to obtain a viscous oil. The oil was dissolved in 100 ml of chloroform. The resulting solution was washed with 50 ml of 1 N hydrochloric acid and concentrated under a reduced pressure. The residue was crystallized from ethyl acetate to obtain 11.4 g of the title compound.

| | | | |
|---|---|---|---|
| melting point (°C): | 181-184 | | |
| elemental analysis: | in terms of | | |
| | $C_{30}H_{32}N_3O_7 \cdot \frac{1}{4}H_2O \cdot HCl$ | | |
| | C | H | N |
| calculated (%) | 59.39 | 5.92 | 6.50 |
| observed (%) | 59.60 | 5.77 | 6.55 |

Example 2

2-[4-(p-Fluorobenzoyl)-1-piperidyl]ethyl methyl 2,6-dimethyl-4-(2',3'-dichlorophenyl)-1,4-dihydropyridine-3,5-dicarboxylate hydrochloride

1.9 g of o,m-dichlorobenzaldehyde, 1,3 g of methyl 3-aminocrotonate, 3.8 g of 2-[4-(p-fluorobenzoyl)-1-piperidyl]ethyl acetoacetate and 30 ml of isopropanol were mixed and heated under reflux for 6 hours.

The reaction mixture was concentrated under a reduced pressure. The residue was purified by silica gel chromatography (with an eluent consisting of chloroform and ethanol in a ratio by volume of 20 to 1) to obtain a viscous oil. The oil was dissolved in 50 ml of chloroform. The resulting solution was washed with 20 ml of 1N hydrochloric acid and concentrated under a reduced pressure. The residue was crystallized from ethyl acetate to obtain 1.3 g of the title compound.

| | | | |
|---|---|---|---|
| melting point (°C): | 174-177 | | |
| elemental analysis: | in terms of | | |
| | $C_{30}H_{31}N_2O_5Cl_2F \cdot HCl$ | | |
| | C | H | N |
| calculated (%) | 57.57 | 5.15 | 4.48 |
| observed (%) | 57.15 | 5.37 | 4.43 |

Example 3

2-[4-(p-Fluorobenzoyl)-1-piperidyl]ethyl ethyl 2,6-dimethyl-4-(2',3'-dichlorophenyl)-1,4-dihydropyridine-3,5-dicarboxylate hydrochloride

4.1 g of 2-[4-(p-fluorobenzoyl)-1-piperidyl]ethyl 2-(o,m-dichlorobenzylidene)acetoacetate, 1.3 g of ethyl 3-aminocrotonate and 30 ml of isopropanol were mixed and heated under reflux for 5 hours.

The reaction mixture was concentrated under a reduced pressure. The residue was purified by silica gel chromatography (with an eluent consisting of chloroform and ethanol in a ratio by volume of 20 to 1) to obtain a viscous oil.

The oil was dissolved in 50 ml of chloroform. The resulting solution was washed with 20 ml of 1N hydrochloric acid and concentrated under a reduced pressure.

The residue was crystallized from ethyl acetate to obtain 1.7 g of the title compound.

melting point (°C): 215 to 218

elemental analysis: in terms of $C_{58}H_{33}N_2O_5Cl_2F\cdot HCl$

| | C | H | N |
|---|---|---|---|
| calculated (%) | 58.64 | 5.24 | 4.41 |
| observed (%) | 58.17 | 5.31 | 4.40 |

Example 4

2-[4-(p-Fluorobenzoyl)-1-piperidyl]ethyl ethyl 2,6-dimethyl-4-(o-chlorophenyl)-1,4-dihydropyridine3,5-dicarboxylate hydrochloride

4.7 g of 2-[4-(p-fluorobenzoyl)-1-piperidyl]ethyl 2-(o-chlorobenzylidene)acetoacetate, 1.5 g of ethyl 3-aminocrotonate and 30 ml of isopropanol were mixed and heated under reflux for 6 hours.

The reaction mixture was concentrated under a reduced pressure. The residue was purified by silica gel chromatography (with an eluent consisting of chloroform and ethanol in a ratio by volume of 20 to 1) to obtain a viscous oil.

The oil was dissolved in 50 ml of chloroform. The resulting solution was washed with 20 ml of 1N hydrochloric acid and concentrated under a reduced pressure.

The residue was crystallized from ethyl acetate to obtain 1.5 g of the title compound.

melting point (°C): 149 to 152

elemental analysis: in terms of $C_{31}H_{32}N_2O_5ClF\cdot HCl$

| | C | H | N |
|---|---|---|---|
| calculated (%) | 61.49 | 5.66 | 4.63 |
| observed (%) | 61.26 | 5.73 | 4.69 |

Example 5

2-[4-(p-Fluorobenzoyl)-1-piperidyl]ethyl 2-methoxyethyl 2,6-dimethyl-4-(m-nitrophenyl)-1,4-dihydropyridine-3,5-dicarboxylate hydrochloride

2.0 g of 2-methoxyethyl 2-(m-nitrobenzylidene)-acetoacetate, 2.3 g of 2-[4-(p-fluorobenzoyl)-1-piperidyl]ethyl acetoacetate, 0.6 g of 28% aqueous ammonia and 30 ml of isopropanol were mixed and heated at 120°C for 12 hours.

After the completion of the reaction, the reaction mixture was poured into 50 ml of water and extracted with 50 ml of chloroform. The extract was concentrated under a reduced pressure. The resulting residue was purified by silica gel chromatography (with an eluent consisting of chloroform and ethanol in a ratio by volume of 20 to 1) to obtain a viscous oil.

The oil was dissolved in 50 ml of chloroform. The resulting solution was washed with 20 ml of 1N hydrochloric acid and concentrated under a reduced pressure.

The residue was crystallized from ethyl acetate to obtain 0.5 g of the title compound.

| | | | |
|---|---|---|---|
| melting point (°C): | 189 - 192 | | |
| elemental analysis: | in terms of $C_{32}H_{36}N_3O_7F \cdot H_2O \cdot HCl$ | | |
| | C | H | N |
| calculated (%) | 59.39 | 5.92 | 6.50 |
| observed (%) | 59.48 | 5.72 | 6.55 |

Referential Example

The 2-[4-(p-fluorobenzoyl)-1-piperidyl]ethyl acetoacetate used in Example 1 as a starting material was prepared as follows:

290g of 2-[4-(p-fluorobenzoyl)-1-piperidyl]-ethanol was mixed with toluene. The resulting mixture was heated at 45°C. 103 g of diketene was dropwise added to the mixture over 20 minutes. After the completion of the dropwise addition, the resulting mixture was heated at 45°C for 1 hour.

After the completion of the reaction, the reaction mixture was washed with 300 ml of water and concentrated under a reduced pressure. The residue was purified by silica gel chromatography (with an eluent consisting of chloroform and ethanol in a ratio by volume of 20 to 1) to obtain 315 g of 2-[4-(p-fluorobenzoyl)-1-piperidyl]ethyl acetoacetate as an oil having the following characteristics:

∘ NMR ( $CDCl_3$ )

$CH_3$ 2.25 (s, 3H)

$\underset{\sim}{CH_2}N$ 2.65 (t, 2H)

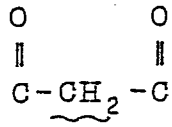 3.44 (s, 2H)

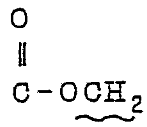 4.25 (t, 2H)

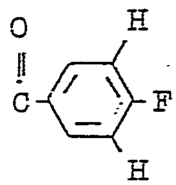 7.11 (t, 2H)

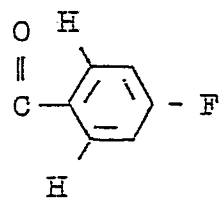 7.95 (dd, 2H)

Examples 6 to 29

The following compounds were synthesized according to the same procedure as the one described in Example 1 or 2. The NMR spectrum (CDCl₃) of each compound (free form) will also be shown below.

Example 6:

2-[4-(p-fluorobenzoyl)-1-piperidyl]ethyl methyl 2,6-dimethyl-4-(o-nitrophenyl)-1,4-dihydropyridine-3,5-dicarboxylate

NMR ($CDCl_3$)

2.29 (3H, s), 2.35 (3H, s), 2.58 (2H, t),

3.57 (3H, s), 4.17 (2H, t), 5.80 (1H, s),

5.85 (1H, s), 7.95 (2H, dd)

Example 7:

2-[4-(p-fluorobenzoyl)-1-piperidyl]ethyl ethyl 2,6-dimethyl-4-(o-nitrophenyl)-1,4-dihydropyridine-3,5-dicarboxylate

NMR ($CDCl_3$)

2.30 (3H, s), 2.32 (3H, s), 2.58 (2H, t), 5.73

(1H, s), 5.80 (1H, s), 7.95 (2H, dd), 1.17 (3H, t)

Example 8:

2-[4-(p-fluorobenzoyl)-1-piperidyl]ethyl methyl 2,6-dimethyl-4-(m-trifluoromethylphenyl)-1,4-dihydropyridine-3,5-dicarboxylate

NMR ($CDCl_3$)

2.30 (3H, s), 2.38 (3H, s), 2.48 (2H, t),

3.62 (3H, s), 4.17 (2H, t), 6.29 (1H, s),

5.06 (1H, s), 7.95 (2H, dd)

Example 9:

3-[4-(p-fluorobenzoyl)-1-piperidyl]propyl methyl 2,6-dimethyl-4-(o-nitrophenyl)-1,4-dihydropyridine-3,5-dicarboxylate

NMR ($CDCl_3$)

2.32 (3H, s), 2.38 (3H, s), 2.52 (2H, t)

3.57 (3H, 2), 6.17 (1H, s), 5.75 (1H, s),

7.94 (2H, dd)

Example 10:

6-[4-(p-fluorobenzoyl)-1-piperidyl]hexyl methyl 2,6-dimethyl-4-(o-nitrophenyl)-1,4-dihydropyridine-3,5-dicarboxylate

NMR ($CDCl_3$)

2.28 (3H, s), 2.35 (3H, s), 2.61 (2H, t),

3.50 (3H, s), 3.98 (2H, t), 6.20 (1H, s),

5.70 (1H, s), 7.90 (2H, dd)

Example 11:

2-[4-(hydroxy-p-fluorophenylmethyl)-1-piperidyl]ethyl methyl 2,6-dimethyl-4-(m-nitrophenyl)-1,4-dihydropyridine-3,5-dicarboxylate

NMR ($CDCl_3$)

2.32 (6H, s), 2.50 (2H, t), 3.60 (3H, s),

4.10 (2H, t), 5.75 (1H, s), 5.20 (1H, s),

4,31 (1H, d)

Example 12:

2-[4-(nitroyx-p-fluorophenylmethyl)-1-piperidyl]-ethyl methyl 2,6-dimethyl-4-(m-nitrophenyl)-1,4-dihydropyridine-3,5-dicarboxylate

NMR ($CDCl_3$)

2.30 (3H, s), 2.36 (3H, s), 2.47 (2H, t),

3.57 (3H, s), 4.06 (2H, t), 5.75 (1H, s),

5.23 (1H, s), 4.26 (1H, d)

Example 13:

2-[4-(p-fluorobenzoyl)-1-piperidyl]ethyl methyl 2,6-dimethyl-4-(m,p-dichlorophenyl)-1,4-dihydropyridine-3,5-dicarboxylate

NMR ($CDCl_3$)

2.31 (6H, s), 2.50 (2H, t), 3.54 (3H, s),

4.07 (4H, t), 5.95 (1H, s), 5.23 (1H, s),

7.92 (2H, dd)

Example 14:

2-[4-(p-fluorobenzoyl)-1-piperidyl]ethyl methyl 2,6-dimethyl-4-(o-chlorophenyl)-1,4-dihydropyridine-3,5-dicarboxylate

NMR ($CDCl_3$)

2.33 (3H, s), 2.58 (3H, s), 2.50 (2H, t),

3.58 (3H, s), 4.05 (2H, t), 5.40 (1H, s),

5.28 (1H, s), 7.96 (2H, dd)

Example 15

2-[4-(p-fluorobenzoyl)-1-piperidyl]ethyl methyl 2,6-dimethyl-4-(o-bromophenyl)-1,4-dihydropyridine-3,5-dicarboxylate

NMR ($CDCl_3$

2.32 (3H, s), 2.35 (3H, s), 2.60 (2H, t),

3.62 (3H, 2), 4.20 (2H, t), 5.57 (1H, s),

5.35 (1H, s), 7.96 (2H, dd)

Example 16:

2-[4-(p-fluorobenzoyl)-1-piperidyl]ethyl methyl 2,6-dimethyl-4-(p-chlorophenyl)-1,4-dihydropyridine-3,5-dicarboxylate

NMR ($CDCl_3$)

2.30 (3H, s), 2.34 (3H, s), 2.58 (2H, t),

3.62 (3H, s), 4.17 (2H, t), 5.77 (1H, s),

4.97 (1H, s), 7.95 (2H, dd)

Example 17:

2-[4-(p-fluorobenzoyl)-1-piperidyl]ethyl methyl 2,6-dimethyl-4-(o,p-dichlorophenyl)-1,4-dihydropyridine-3,5-dicarboxylate

NMR ($CDCl_3$)

2.30 (6H, s), 2.58 (2H, t), 3.60 (3H, s),

4.13 (2H, t), 5.62 (1H, s), 5.32 (1H, s),

7.96 (2H, dd)

Example 18

2-[4-(p-fluorobenzoyl)-1-piperidyl]ethyl methyl 2,6-dimethyl -4-(2'-chloro-5'-nitrophenyl)-1,4-dihydropyridine-3,5-dicarboxylate

NMR ($CDCl_3$)

2.40 (6H, s), 2.65 (2H, t), 3.67 (3H, s),

4.23 (2H, t), 6.70 (1H, s), 5.52 (1H, s),

7.94 (2H, dd)

Example 19:

2-[4-(p-fluorobenzoyl)-1-piperidyl]ethyl methyl 2,6-dimethyl-4-(2', 6'-dichlorophenyl)-1,4-dihydropyridine-3,5-dicarboxylate

NMR ($CDCl_3$)

2.10 (6H, s), 2.52 (2H, t), 3.52 (3H, s),

4.12 (2H, t), 6.31 (1H, s), 5.91 (1H, s),

7.96 (2H, dd)

Example 20:

2-[4-(p-fluorobenzoyl)-1-piperidyl]ethyl methyl 2,6-dimethyl-4-(m-chlorophenyl)-1,4-dihydropyridine-3,5-dicarboxylate

NMR ($CDCl_3$)

2.34 (3H, s), 2.36 (3H, s), 2.61 (2H, t),

3.65 (3H, s), 4.21 (2H, t), 5.77 (1H, s),

5.00 (1H, s), 7.97 (2H, dd)

Example 21:

2-[4-(p-fluorobenzoyl)-1-piperidyl]ethyl methyl 2,6-dimethyl-4-(o-cyanophenyl)-1,4-dihydropyridine-3,4-dicarboxylate

NMR ($CDCl_3$)

2.37 (3H, s), 2.39 (3H, s), 2.64 (2H, t),

3.65 (3H, s), 4.21 (2H, t), 6.76 (1H, s),

5.34 (1H, s), 7.98 (2H, dd)

Example 22:

2-[4-(p-fluorobenzoyl)-1-piperidyl]ethyl methyl 2,6-dimethyl-4-naphthyl-1,4-dihydropyridine-3,5-dicarboxylate

NMR ($CDCl_3$)

2.27 (3H, s), 2.34 (3H, s), 2.67 (2H, t),

3.44 (3H, s), 4.03 (2H, t), 6.62 (1H, s),

5.83 (1H, s), 7.95 (2H, dd)

Example 23:

2-[4-(p-fluorobenzoyl)-1-piperidyl]ethyl methyl 2,6-dimethyl-4-(3-pyridyl)-1,4-dihydropyridine-3,5-dicarboxylate

NMR ($CDCl_3$)

2.36 (3H, s), 2.39 (3H, s), 2.62 (2H, t),

3.64 (3H, s), 4.19 (2H, t), 6.72 (1H, s),

5.04 (1H, s), 7.98 (2H, dd)

Example 24:

2-[4-(p-fluorobenzoyl)-1-piperidyl]ethyl methyl 2,6-dimethyl-4-(2'-chloro-4',5'-methylenedioxyphenyl)-1,4-dihydropyridine-3,5-dicarboxylate

NMR ($CDCl_3$)

2.29 (6H, s), 2.68 (2H, t), 3.64 (3H, s),

4,30 (2H, t), 6.45 (1H, s), 5.89 (1H, s),

7.99 (2H, dd)

Example 25:

2-[4-(p-fluorobenzoyl)-1-piperidyl]ethyl methyl 2,6-dimethyl-4-(m-nitro-p-chlorophenyl)-1,4-dihydropyridine-3,5-dicarboxylate

NMR ($CDCl_3$)

2.36 (3H, s), 2.38 (3H, s), 2.60 (2H, t),

3.66 (3H, s), 4.19 (2H, t), 5.95 (1H, s),

5.03 (1H, s), 7.98 (2H, dd)

Example 26:

2-[4-(p-fluorobenzoyl)-1-piperidyl]ethyl methyl 2,6-dimethyl-4-(2-furyl)-1,4-dihydropyridine-3,5-dicarboxylate

NMR ($CDCl_3$)

2.25 (6H, s), 2.67 (2H, t), 3.66 (3H, s),

4.30 (2H, t), 6.00 (1H, s), 5.05 (1H, s),

7.96 (2H, dd)

Example 27:

2-[4-(p-fluorobenzoyl)-1-piperidyl]ethyl methyl 2,6-dimethyl-4-(3-indolyl)-1,4-dihydropyridine-3,5-dicarboxylate

NMR ($CDCl_3$)

2.30 (3H, s), 2.34 (3H, s), 2.57 (2H, t),

3.61 (3H, s), 4.14 (2H, t), 6.12 (1H, s),

5.34 (1H, s), 7.92 (2H, dd)

Example 28:

2-[4-(hydroxy-p-fluorophenylmethyl)-1-piperidyl]ethyl ethyl 2,6-dimethyl-4-(m-chlorophenyl)-1,4-dihydropyridine-3,5-dicarboxylate

NMR ($CDCl_3$)

2.30 (6H, s), 2.54 (2H, t), 5.78 (1H, s),

5.36 (1H, s), 4.33 (1H, d), 1.19 (3H, t)

Example 29

2-methyl-3-methoxycarbonyl-4-(m-nitrophenyl)-5-ethoxycarbonyl-6-[4-(p-fluorobenzoyl)-1-piperidyl]methyl-1,4-dihydropyridine

NMR ($CDCl_3$)

2.29 (3H, s), 2.35 (3H, s), 2.58 (2H, t),

4.17 (2H, t), 5.81 (1H, s), 5.85 (1H, s),

7.95 (2H, dd)

Examples 30 to 32

The following compounds were synthesized according to the same procedure as the one described in Example 3 or 4. The NMR spectrum of each compound ($CDCl_3$) will also be shown below.

Example 30:

2-[4-(p-fluorobenzoyl)-1-piperidyl]ethyl ethyl 2,6-dimethyl-4-(2',3'-dichlorophenyl)-1,4-dihydro pyridine-3,5-dicarboxylate

NMR ($CDCl_3$)

2.29 (3H, s), 2.31 (3H, s), 2.58 (2H, t),

5.92 (1H, s), 5.47 (1H, s), 7.98 (2H, dd)

1.18 (3H, t)

Example 31:

2-[4-(p-fluorobenzoyl)-1-piperidyl]ethyl ethyl 2,6-dimethyl-4-(o-chlorophenyl)-1,4-dihydropyridine-3,5-dicarboxylate

NMR ($CDCl_3$)

2.33 (6H, s), 2.62 (2H, t), 5.82 (1H, s),

5.40 (1H, s), 7.96 (2H, dd), 1.20 (3H, t)

Example 32:

2-[4-(p-fluorobenzoyl)-1-piperidyl]ethyl methyl 2,6-dimethyl-4-(o-fluorophenyl)-1,4-dihydropyridine-3,5-dicarboxylate

NMR ($CDCl_3$)

2.30 (3H, s), 2.32 (3H, s), 3.66 (3H, s),

4.30 (2H, t), 5.45 (1H, t), 5.89 (1H, s),

7.96 (2H, dd)

Examples 33 to 35

The following compounds were synthesized according to the same procedure as the one described in Example 5. The NMR spectrum of each compound ($CDCl_3$) will also be shown below.

Example 33:

2-[4-(p-fluorobenzoyl)-1-piperidyl]ethyl 2-[4-(p-fluorobenzoyl)-1-piperidyl]ethyl 2,6-dimethyl-4-(m-nitrophenyl)-1,4-dihydropyridine-3,5-dicarboxylate

NMR ($CDCl_3$)

2.38 (6H, s), 2.58 (4H, t), 4.18 (4H, t),

6.30 (1H, s), 5.07 (1H, s), 7.8 -8.1 (4H, m),

Example 34:

2-methyl-3-[2-(4-p-fluorobenzoyl)-1-piperidylethoxycarbonyl-4-(m-nitrophenyl)-5(1H, 4H, 6H, 7H, 8H)-pentahydroquinolinone

NMR ($CDCl_3$)

3.39 (3H, s), 9.56 (1H, s), 5.02 (1H, s),

7.99 (2H, dd)

Example 35

2-[4-(p-fluorobenzoyl)-1-piperidyl]ethyl 2-methoxyethyl 2,6-dimethyl-4-(m-nitrophenyl-3,5-dicarboxylate

NMR ($CDCl_3$)

2.37 (3H, s), 2.39 (3H, s), 2.61 (2H, t),

6.03 (1H, s), 5.14 (1H, s), 7.99 (2H, dd),

3.36 (1H, s)

**Claims**

1. A 1,4-dihydropyridine derivative represented by the general formula:

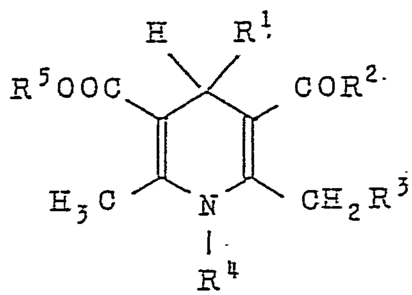

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom consisting an aryl group; $R^2$ stands for a lower alkoxy group, a group represented by the general formula:

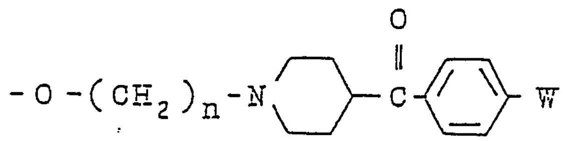

(wherein n stands for a positive number of 2 to 6 and W stands for a hydrogen or halogen atom or a lower alkoxy group) or a group represented by the general formula:

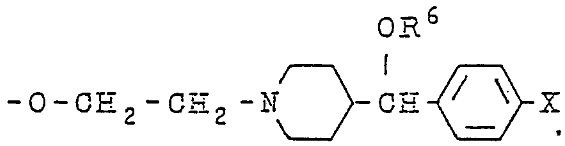

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

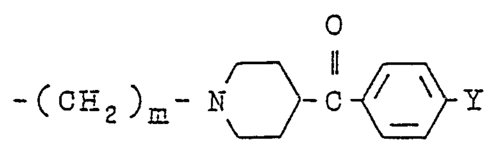

(wherein m stands for a positive number of 0 to 5 and Y stands for a hydrogen or halogen atom or a lower alkoxy group)];

$R^5$ stands for a lower alkyl or lower alkoxy-lower alkyl group, a group represented by the general formula:

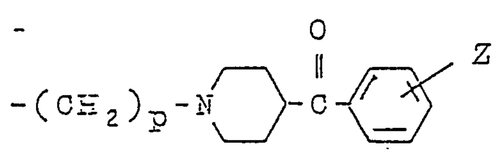

(wherein p stands for a positive number of 1 to 6 and Z stands for a hydrogen or halogen atom or a lower alkoxy group) or a group represented by the general formula:

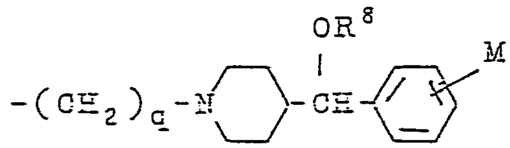

(wherein q stands for a positive number of 1 to 6 and M stands for a hydrogen or halogen atom or a lower alkoxy group),

or a pharmaceutically acceptable salt thereof.

2. A process for the preparation of 1,4-dihydropyridine derivative represented by the general formula

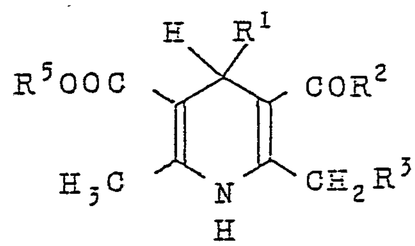

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom constituting an aryl group; $R^2$ stands for a lower alkoxy group, a group represented by the general formula:

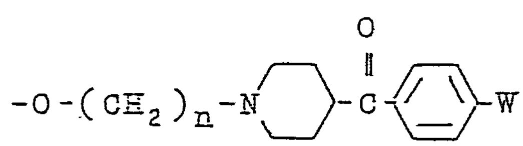

(wherein n stands for a positive number of 2 to 6 and W stands or a hydrogen or halogen atom or a lower alkoxy group) or a group represented by the general formula:

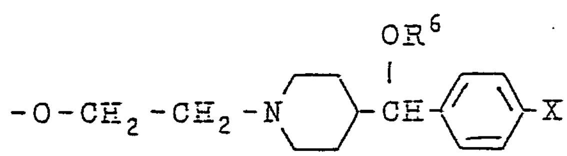

(wherein $R^6$ stands for a hydrogen atom or a nitro group and X stnads for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^5$ stands for a lower alkyl or lower alkoxy lower alkyl group, a group represented by the general formula:

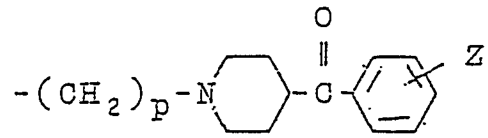

(wherein p stands for a positive number of 1 to 6 and Z stands for a hydrogen or halogen atom or a lower alkoxy group) or a group represented by the general formula:

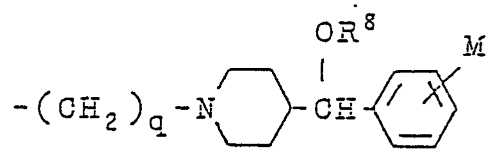

(wherein q stands for a positive number of 1 to 6 and M stands for a hydorgen or halogen atom or a lower alkoxy group),

which comprises reacting

1 a compound represented by the general formula:

$R^1$-CHO

wherein $R^1$ is as defined above,

with

2 an acetoacetate derivative represented by the general formula:

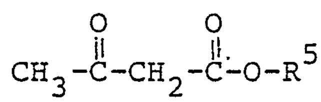

wherein $R^5$ is as defined above,

and

3 an enaminocarboxylate derivative represented by the general formula:

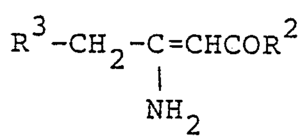

wherein $R^2$ and $R^3$ are as defined above.

3. A process for the preparation of 1,4-dihydropyridine derivative represented by the general formula:

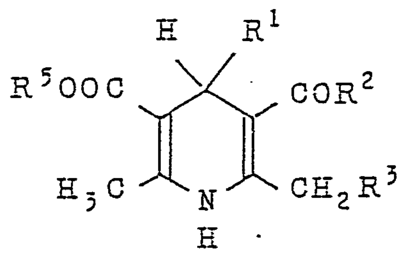

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom consistituting an aryl group; $R^2$ stands for a lower alkoxy group, a group represented by the general formula:

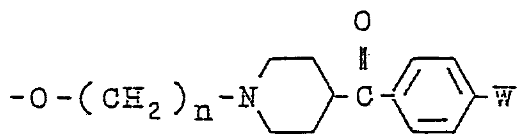

(wherein n stands for a positive number of 2 to 6 and W stands for a hydrogen or halogen atom or a lower alkoxy group) or a group represented by the general formula:

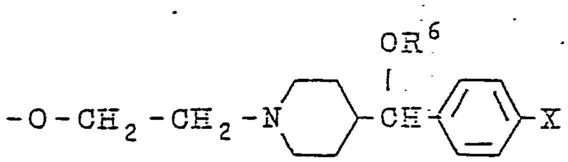

(wherein $R^6$ stands for a hydrogen atom or a nitro group and X stands for a hydrogen or halogen atom or a lower alkoxy group); $R^3$ stands for a hydrogen atom or a 4-p-(fluorobenzoyl)-1-piperidyl group, or alternatively, $R^2$ and $R^3$ may together form a ring; $R^5$ stands for a lower alkyl or lower alkoxy lower alkyl group, a group represented by the general formula:

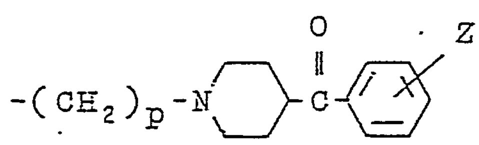

(wherein p stands for a positive number of 1 to 6 and Z stands for a hydrogen or halogen atom or a lower alkoxy group) or a group represented by the general formula:

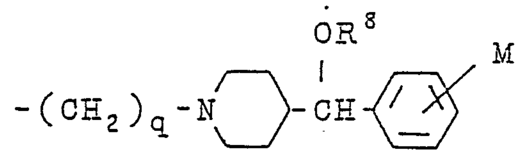

(wherein q stands for a positive number of 1 to 6 and M stands for a hydorgen or halogen atom or a lower alkoxy group;

which comprises reacting a benzylideneacetoacetate derivative represented by the general formula:

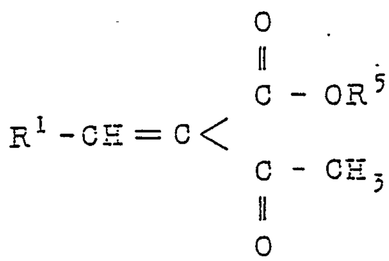

wherein $R^1$ and $R^5$ are as defined above, with an enaminocarboxylate derivative represented by the general formula:

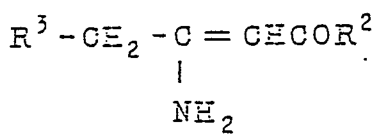

wherein $R^2$ and $R^3$ are as defined above.

4. A process for the preparation of a 1,4-dihydropyridine derivative represented by the general formula

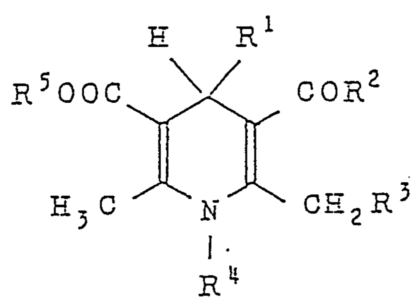

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom constituting an aryl group; $R^2$ stands for a lower alkoxy group, a group represented by the general formula:

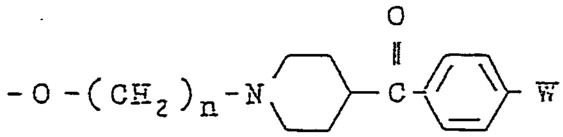

(wherein n stands for a positive number of 2 to 6 and W stands for a hydrogen or halogen atom or a lower alkoxy group) or a group represented by the general formula:

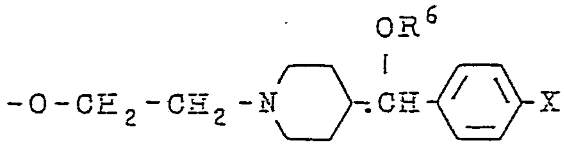

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring:

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

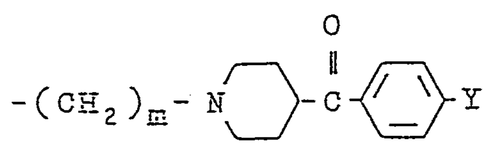

(wherein m stands for a positive number of 0 to 5 and Y stands for a hydrogen or halogen atom or a lower alkoxy group)];

$R^5$ stands for a lower alkyl or lower alkoxy-lower alkyl group, a group represented by the general formula:

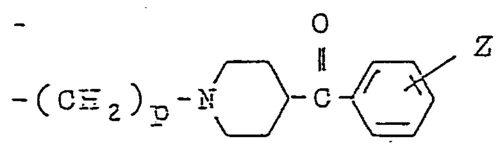

(wherein p stands for a positive number of 1 to 6 and Z stands for a hydrogen or halogen atom or a lower alkoxy group) or a group represented by the general formula:

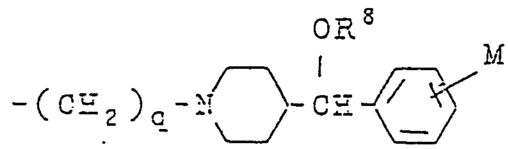

(wherein q stands for a positive number of 1 to 6 and M stands for a hydrogen or halogen atom or a lower alkoxy group),

or a pharmaceutically acceptable salt thereof, which comprises condensing a compound represented by the general formula:

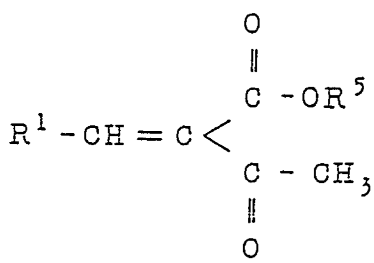

(VI)

wherein $R^1$ and $R^5$ are as defined above, with a compound represented by the general formula:

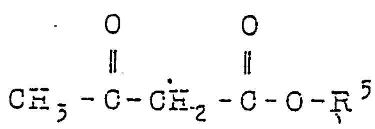

(III)

wherein $R^5$ is as defined above

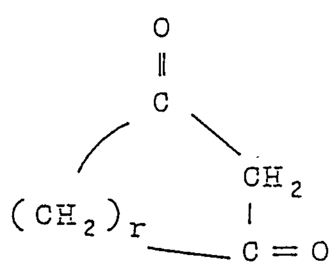

(VII)

wherein r stands for a positive number of 2 to 4, and an aqueous ammonia (VIII) or a compound represented by the general formula:

$H_2N-(CH_2)_s-R^9$ (IX)

wherein s stands for a positive number of 0 to 6 and $R^9$ stands for a hydrogen atom or a lower alkyl, phenylalkyl, lower alkoxy alokoxy or phenyl group, a group represented by the formula

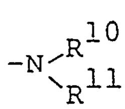

(wherein $R^{10}$ and $R^{11}$ may be the same or different from each other and each stands for a lower alkyl group) or a group represented by the formula

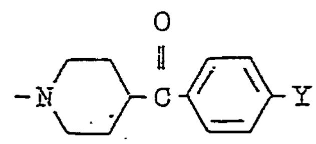

(wherein Y stands for a hydrogen or halogen atom or a lower alkoxy group).

5. A medicine for treatment or prevention of cardiopathy containing, as an active principle, 1,4-dihydropyridine derivative represented by the general formula:

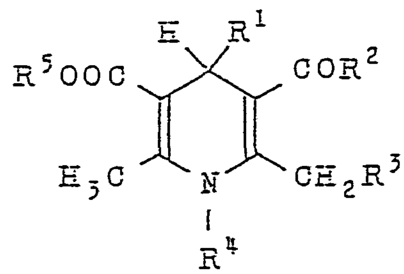

wherein $R^1$ stands for an aryl or heteroaryl group which may be substituted with a lower alkyl, lower alkoxy, halogen, nitro, trofluoromethyl or cyano group or may form an alkylenedioxy group together with an adjacent carbon atom constituting an aryl group; $R^2$ stands for a lower alkoxy group, a group represented by the general formula:

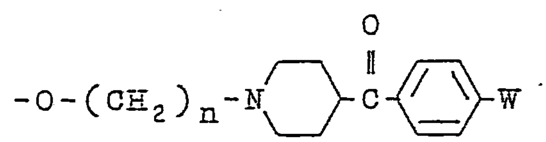

(wherein n stands for a positive number of 2 to 6 and W stands for a hydrogen or halogen atom or a lower alkoxy group) or a group represented by the general formula:

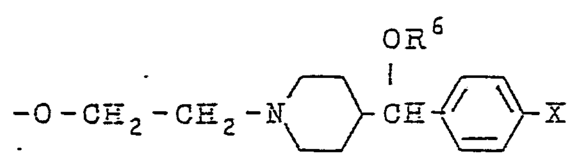

(wherein $R^6$ stands for a hydrogen atom or a nitro group and X stands for a hydorgen or halogen atom or a lower alkoxy group);

$R^3$ stands for a hydrogen atom or a 4-p-fluorobenzoyl)-1-piperidyl group, or alternatively, $R^2$ and $R^3$ may together form a ring;

$R^4$ stands for a hydrogen atom, a lower alkyl or phenylalkyl group or a group represented by the

formula $-CH_2-L$ [wherein L stands for a group of $-OR^7$ (wherein $R^7$ stands for a lower alkyl group) or a group represented by the general formula:

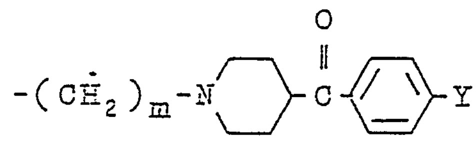

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

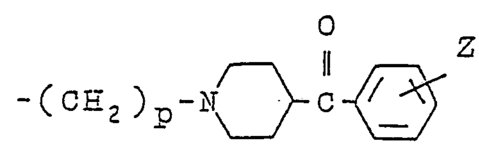

(wherein p stands for a positive number of 1 to 6 and Z stands for a hydrogen or halogen atom or a lower alkoxy group) or a group represented by the gênerai formula:

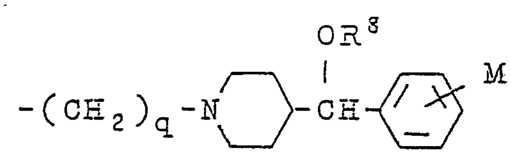

(wherein q stands for a positive number of 1 to 6 and M stands for a hydrogen or halogen atom or a lower alkoxy group),

or its pharmaceutically acceptable salt.

6. A medicine based on antihypertensive or coronary vasodilating action containing, as an active principle, 1,4-dihydropyridine derivative represented by the general formula:

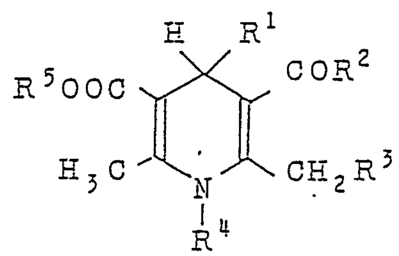

wherein $R^1$ stands for an aryl or heteroaryl group which may be substituted with a lower alkyl, lower alkoxy, halogen, nitro, trifluoromethyl or cyano group or may form an alkylene dioxy group together with an adjacent carbon atom constituting an aryl group; $R^2$ stands for a lower alkoxy group, a group represented by the general formula:

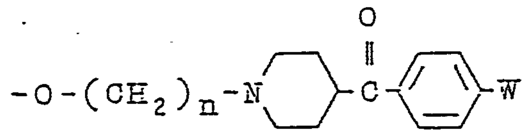

(wherein n stands for a positive number of 2 to 6 and W stands for a hydrogen or halogen atom or a lower alkoxy group) or a group repressented by the general formula:

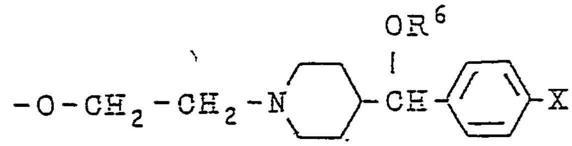

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl- group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

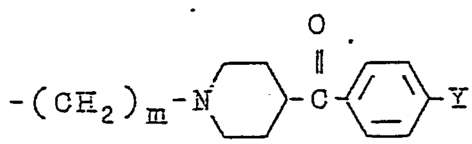

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

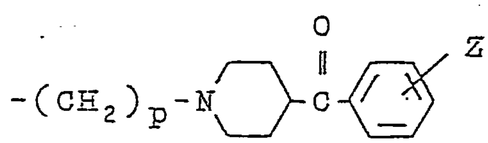

(wherein p stands for a positive number of 1 to 6 and Z stands for a hydrogen or halogen atom or a lower alkoxy group) or a group represented by the general formula:

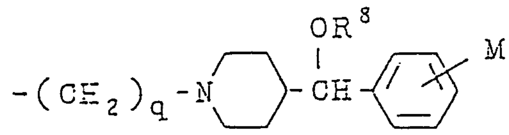

(wherein q stands for a positive number of 1 to 6 and M stands for a hydrogen or halogen atom or a lower alkoxy group),

or its pharmaceutically acceptable salt.

7. The use of 1,4-dihydropyridine derivative according to claim 1 as well as pharmaceutically acceptable salts thereof for the preparation of a drug for the treatment or prevention of cardiopathy.

8. The use of 1,4-dihydropyridine derivative according to claim 1 as well as pharmaceutically acceptable salts thereof for the preparation of a drug for the treatment of brain circulation failure or hypertension.

Claim(s) for contracting state : AT

1. A process for the preparation of a 1,4-dihydropyridine derivative represented by the general formula:

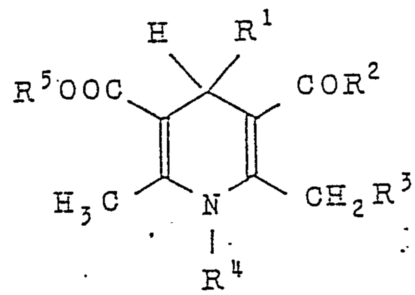

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom constituting an aryl group; $R^2$ stands for a lower alkoxy group, a group represented by the general formula:

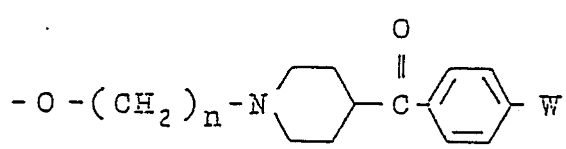

(wherein n stands for a positive number of 2 to 6 and W stands for a hydrogen or halogen atom or a lower alkoxy group) or a group represented by the general formula:

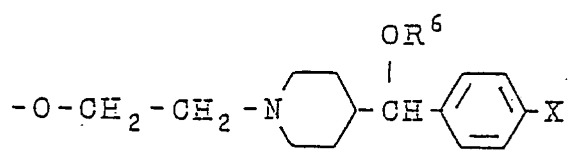

(wherein $R^6$ stands for a hydrogen atom or a nitro group and X' stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

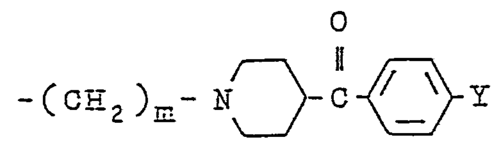

(wherein m stands for a positive number of 0 to 5 and Y stands for a hydrogen or halogen atom or a lower alkoxy group)];

$R^5$ stands for a lower alkyl or lower alkoxy-lower alkyl group, a group represented by the general formula:

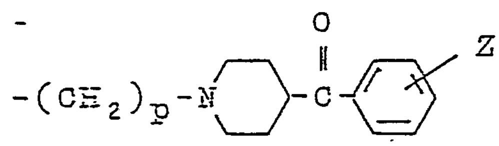

(wherein p stands for a positive number of 1 to 6 and Z stands for a hydrogen or halogen atom or a lower alkoxy group) or a group represented by the general formula:

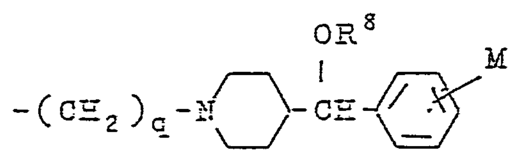

(wherein q stands for a positive number of 1 to 6 and M stands for a hydrogen or halogen atom or a lower alkoxy group),

or a pharmaceutically acceptable salt thereof, which comprises condensing a compound represented by the general formula:

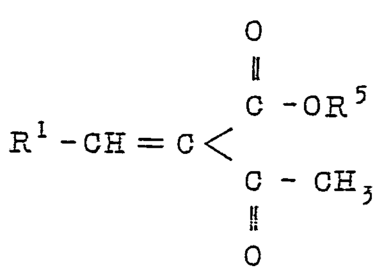

(VI)

wherein $R^1$ and $R^5$ are as defined above, with a compound represented by the general formula:

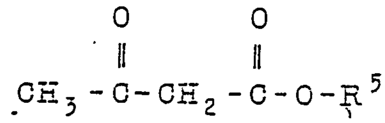

(III)

wherein $R^5$ is as defined above

or

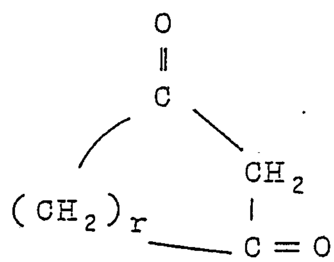

(VII)

wherein r stands for a positive number of 2 to 4, and an aqueous ammonia (VIII) or a compound represented by the general formula:

$H_2N-(CH_2)_s-R^9$ (IX)

wherein s stands for a positive number of 0 to 6 and $R^9$ stands for a hydrogen atom or a lower alkyl, phenylalkyl, lower alkoxy or phenyl group, a group represented by the formula

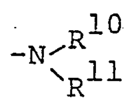

(wherein $R^{10}$ and $R^{11}$ may be the same or different from each other and each stands for a lower alkyl group) or a group represented by the formula

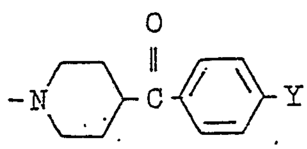

(wherein Y stands for a hydrogen or halogen atom or a lower alkoxy group).

2. A process for the preparation of 1,4-dihydropyridine derivative represented by the general formula

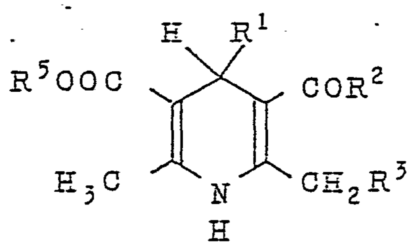

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom constituting an aryl group; $R^2$ stands for a lower alkoxy group; a group represented by the general formula:

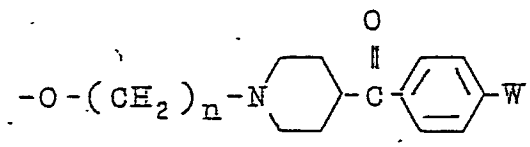

(wherein n stands for a positive number of 2 to 6 and W stands for a hydrogen or halogen atom or a lower alkoxy group) or a group represented by the general formula:

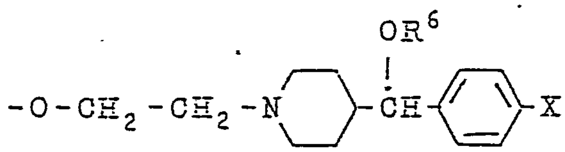

(wherein $R^6$ stands for a hydrogen atom or a nitro group and X stnads for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^5$ stands for a lower alkyl or lower alkoxy lower alkyl group, a group represented by the general formula:

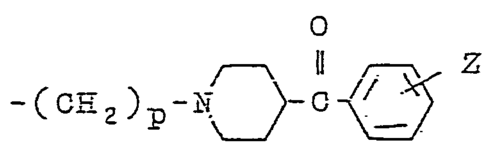

(wherein p stands for a positive number of 1 to 6 and Z stands for a hydrogen or halogen atom or a lower alkoxy group) or a group represented by the general formula:

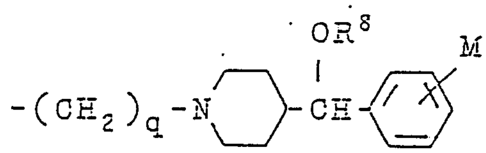

(wherein q stands for a positive number of 1 to 6 and M stands for a hydrogen or halogen atom or a lower alkoxy group),

which comprises reacting

1 a compound represented by the general formula:

$R^1$-CHO

wherein $R^1$ is as defined above,

with

2 an acetoacetate derivative represented by the general formula:

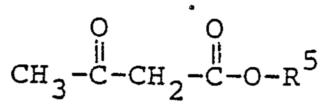

wherein $R^5$ is as defined above,

and

3 an enaminocarboxylate derivative represented by the general formula:

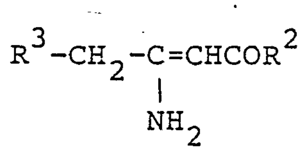

wherein $R^2$ and $R^3$ are as defined above.

3. A process for the preparation of 1,4-dihydropyridine derivative represented by the general formula:

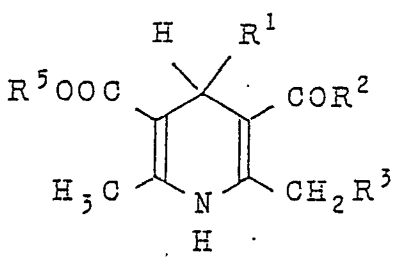

wherein $R^1$ stands for an aryl or heteroaryl group which may be substituted with a lower alkyl, lower alkoxy, halogen, nitro, trifluoromethyl or cyano group or may form an alkylenedioxy group together with an adjacent carbon atom consistituting an aryl group; $R^2$ stands for a lower alkoxy group, a group represented by the general formula:

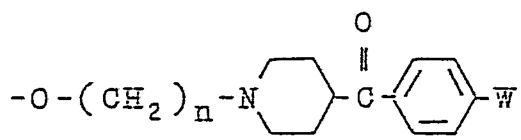

(wherein n stands for a positive number of 2 to 6 and W stands for a hydrogen or halogen atom or a lower alkoxy group) or a group represented by the general formula:

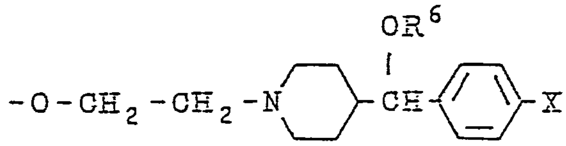

(wherein $R^6$ stands for a hydrogen atom or a nitro group and X stands for a hydrogen or halogen atom or a lower alkoxy group);

$R^3$ stands for a hydrogen atom or a 4-p--(fluorobenzoyl)-1-piperidyl group, or alternatively, $R^2$ and $R^3$ may together form a ring;

$R^5$ stands for a lower alkyl or lower alkoxy lower alkyl group, a group represented by the general formula:

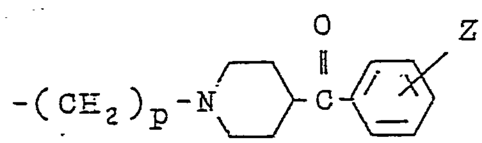

(wherein p stands for a positive number of 1 to 6 and Z stands for a hydrogen or halogen atom or a lower alkoxy group) or a group represented by the general formula:

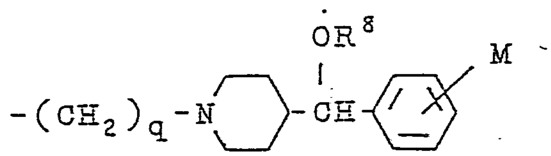

(wherein q stands for a positive number of 1 to 6 and M stands for a hydorgen or halogen atom or a lower alkoxy group;

which comprises reacting a benzylideneacetoacetate derivative represented by the general formula:

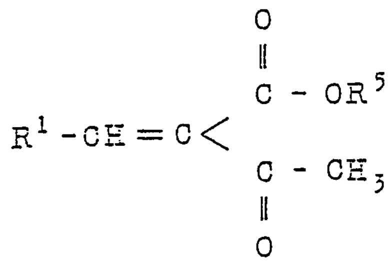

wherein $R^1$ and $R^5$ are as defined above, with an enaminocarboxylate derivative represented by the general formula:

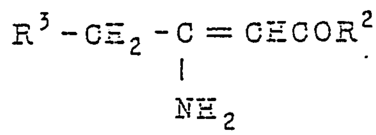

wherein $R^2$ and $R^3$ are as defined above.

4. A process for the preparation of a medicine useful in the treatment or prevention of cardiopathy, comprising the mixing of 1,4-dihydropyridine derivative represented by the general formula

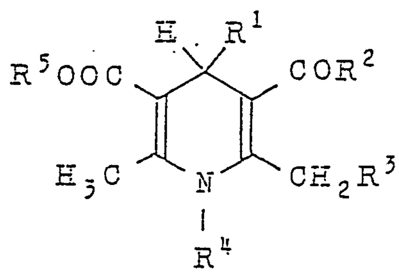

wherein $R^1$ stands for an aryl or heteroaryl group which may be substituted with a lower alkyl, lower alkoxy, halogen, nitro, trofluoromethyl or cyano group or may form an alkylenedioxy group together with an adjacent carbon atom constituting an aryl group; $R^2$ stands for a lower alkoxy group, a group represented by the general formula:

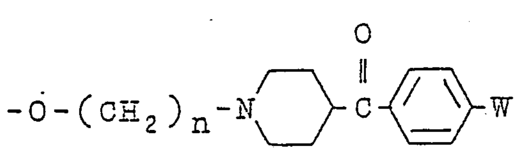

(wherein n stands for a positive number of 2 to 6 and W stands for a hydrogen or halogen atom or a lower alkoxy group) or a group represented by the general formula:

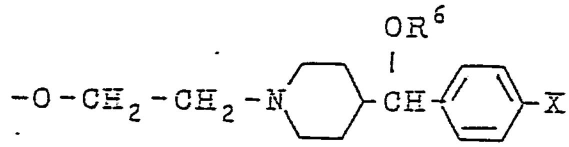

(wherein $R^6$ stands for a hydrogen atom or a nitro group and X stands for a hydrogen or halogen atom or a lower alkoxy group);

$R^3$ stands for a hydrogen atom or a 4-p-(fluorobenzoyl)-1-piperidyl group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

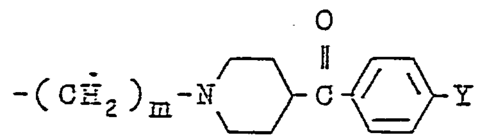

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

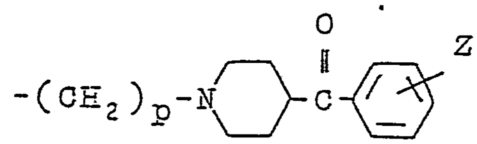

(wherein p stands for a positive number of 1 to 6 and Z stands for a hydrogen or halogen atom or a lower alkoxy group) or a group represented by the general formula:

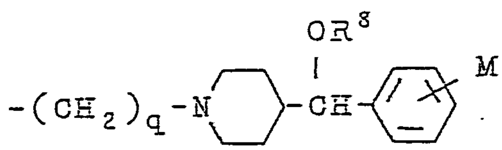

(wherein q stands for a positive number of 1 to 6 and M stands for a hydrogen or halogen atom or a lower alkoxy group),

or its pharmaceutically acceptable salt, with a suitable carrier or diluent.

5. A process for the preparation of a medicine based on antihypertensive or coronary vasodilating action, comprising the mixing of 1,4-dihydropyridine derivative represented by the general formula

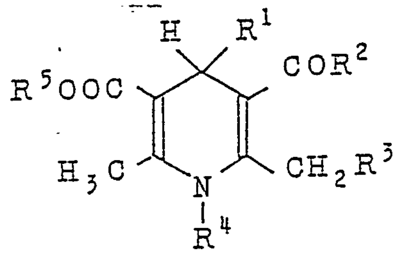

wherein $R^1$ stands for an aryl or heteroaryl group which may be susbstituted with a lower alkyl, lower alkoxy, halogen, nitro, trifluoromethyl or cyano group or may form an alkylene dioxy group together with an adjacent carbon atom constituting an aryl group; $R^2$ stands for a lower alkoxy group, a group represented by the general formula:

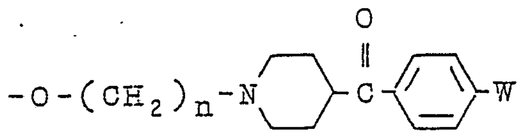

(wherein n stands for a positive number of 2 to 6 and W stands for a hydrogen or halogen atom or a lower alkoxy group) or a group represented by the general formula:

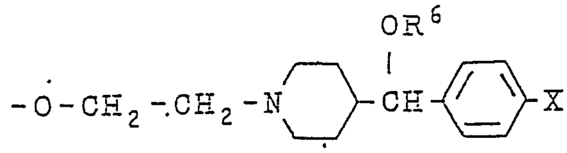

(wherein $R^6$ stands for a hydrogen atom or a nitro group and X stands for a hydrogen or halogen atom or a lower alkoxy group);

$R^3$ stands for a hydrogen atom or a 4-p-fluorobenzoyl)-1-piperidyl group, or alternatively, $R^2$ and $R^3$ may together form a ring;

$R^4$ stands for a hydrogen atom, a lower alkyl or phenylalkyl group or a group represented by the formula $-CH_2-L$ [wherein L stands for a group of $-OR^7$ (wherein $R^7$ stands for a lower alkyl group) or a group represented by the general formula:

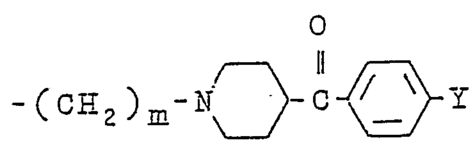

(wherein m stands for a positive number of 0 to 5 and Y stands for a hydrogen or halogen atom or a lower alkoxy group);

$R^5$ stands for a lower alkyl or lower alkoxy lower alkyl group, a group represented by the general formula:

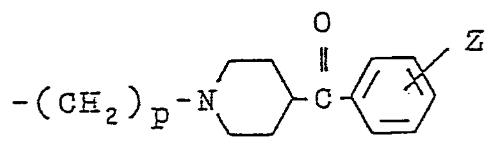

(wherein p stands for a positive number of 1 to 6 and Z stands for a hydrogen or halogen atom or a lower alkoxy group) or a group represented by the general formula:

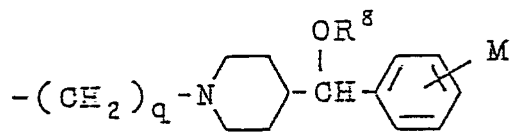

(wherein q stands for a positive number of 1 to 6 and M stands for a hydrogen or halogen atom or a lower alkoxy group),

or its pharmaceutically acceptable salt, with a suitable carrier or diluent.